# EUROPEAN PATENT APPLICATION

(11) **EP 4 083 061 A1**
(43) Date of publication of application: **02.11.2022**
(21) Application number: 21171407.6
(22) Date of filing: 30.04.2021
(51) Int. Cl.: C07K 14/705, C07K 16/18, C12N 15/09, C12N 15/62, C07K 19/00, A61P 13/12, A61K 39/395

(54) **FUSION PROTEINS AND USE THEREOF IN THE TREATMENT OF MEMBRANOUS NEPHROPATHY**

(71) Applicant: Kilometro Rosso SPA, 24126 BERGAMO (IT)
(72) Inventor: PERICO, Luca, 24126 Bergamo (BG) (IT); CASIRAGHI, Federica, 24126 Bergamo (BG) (IT); BENIGNI, Ariela, 24126 Bergamo (BG) (IT); REMUZZI, Giuseppe, 24126 Bergamo (BG) (IT)
(74) Representative: Bianchetti & Minoja SRL

(57) **Abstract**

There are disclosed fusion proteins comprising an anti-CD3 antibody and an autoantigen involved in autoimmune diseases, nucleic acid encoding the same, pharmaceutical composition comprising them and the use thereof for the treatment of autoimmune diseases and particularly of membranous nephropathy.

## Description

The present invention relates to the immunotherapy of autoimmune diseases. Specifically, the invention provides fusion proteins comprising an anti-CD3 antibody and the autoantigen involved in the autoimmune disease. The fusion proteins of the invention are useful for the treatment of autoimmune diseases and particularly of membranous nephropathy. Other aspects of the invention relate to nucleic acid molecules encoding the fusion proteins, expression vectors and pharmaceutical composition thereof.

### Background of the invention

Autoimmunity is a highly heterogeneous pathogenic condition in which the immune system produces antibodies that attack normal body tissues. The causes of self-tolerance loss still remain unknown, although there is growing consensus that autoimmune diseases likely result from the interactions of genetic and environmental factors. To date, 80-100 different autoimmune diseases have been identified and at least 40 additional diseases are suspected of having an autoimmune basis. These diseases have a broad spectrum of clinical presentations and unpredictable courses, depending on the main affected organ toward which pathogenic autoantibodies are directed. Given the chronic nature of these pathogenic conditions, autoimmune diseases can be life-threatening and they are one of the top 10 leading causes of death in female children and women in all age groups up to 64 years.¹ The National Institutes of Health estimates up to 24 million Americans suffer from autoimmune diseases and that the prevalence is rising worldwide.² A recent report by the American Autoimmune Related Diseases Association highlighted that autoimmune diseases imposes an annual direct health care costs in the range of $100 billion.³

Current treatments for autoimmune conditions mainly rely on non-specific and general suppression of the immune cells. However, a major shortcoming of existing therapies is that they destroy not just the specific cells that mistakenly target and attack the body's own healthy tissue, but also the other immune cells that are functioning normally. This leaves the body exposed to infections and other diseases, including cancer. For this reason, targeted suppression of autoreactive immune cells without collateral suppression of normal immunity remains an elusive yet clinically important unmet need in autoimmune diseases.

Advances in the pathophysiology of autoimmune diseases has highlighted the pivotal role of autoreactive B cells in the pathogenesis of autoimmunity. Several attempts have been made to develop safe and effective B-cell targeted therapies in autoimmune diseases. This is the case of the monoclonal antibodies (mAbs) against the CD20 antigen present on B lymphocytes, such as rituximab which was approved by the US Food and Drug Administration for treating non-Hodgkin's lymphoma. The anti-CD20 antibodies bind to and kill B cells either by inducing B cell apoptosis or by triggering Fcγ receptor (FcγR)-mediated antibody-dependent cell-mediated cytotoxicity and/or complement-dependent cytotoxicity of targeted B cells. Rituximab has proved effective in inducing disease remission in several autoimmune conditions, including systemic lupus erythematosus (SLE), multiple sclerosis (MS), pemphigus vulgaris (PV), and membranous nephropathy (MN).⁴⁻⁷

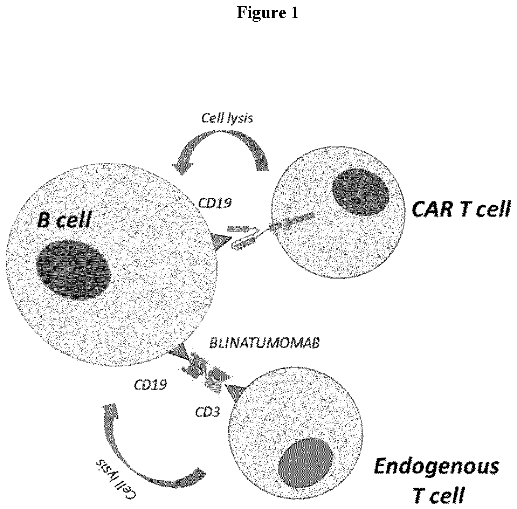

In the field of oncology, chimeric antigen receptor (CAR) technology has revolutionised cancer therapy by providing a novel approach to deplete B cells in a sustained and specific fashion **(****Figure 1****).** A CD19-specific CAR, consisting of an extracellular single-chain variable fragment (scFv) antibody to CD19, fused to T cell cytoplasmic signalling domains, activates T cell cytotoxicity upon contact with CD19⁺ B cells, causing the specific and permanent elimination of B cells and durable remission of leukaemia. Recent evidence has suggested that sustained CD19⁺ B cell depletion by CD19-specific CAR is a highly effective therapy in an experimental model of systemic lupus erythematosus,⁸ thus suggesting that CAR T cells can have a potential therapeutic effect in autoimmune diseases. Similarly, reengineering chimeric antigen receptor T cells have been suggested for targeted therapy of autoimmune disease.9 However, the generation of CAR T cells requires the isolation and engineering of patient-specific T cells, creating extremely long and expensive production times.

Several other biologics have been developed to induce deep depletion of B cells in patients with B cell malignancies, including bi-specific T cell engager antibody (BiTE), such as blinatumomab.¹⁰ Blinatumomab is a 55-kDa BiTE directed to the B cell receptor CD19 and T cell receptor CD3 **(****Figure 1**).¹⁰ When the two cells are linked by the bispecific antibody, this immunological synapse causes the release of inflammatory cytokines, the production of cytolytic proteins, and proliferation of T cells, ultimately resulting in the lysis of the engaged CD19 B cells.^{11,12} All available evidence suggests that BiTEs do not need additional T cell stimuli for redirected cell lysis against malignant B cells.^{11,12} Indeed, the first published studies on BiTEs typically took advantage of unstimulated peripheral human T lymphocytes. However, monovalent binding of the anti-CD3 arm to the CD3 complex by BiTEs does not lead to a full-blown T-cell activation, even at high concentrations. Conversely, optimal T cell activation by BiTEs only occur when BiTEs are arrayed on the surface of a target B cell, redirecting robust T cell lytic activity on B cells without an apparent need for costimulatory and mitogenic T cell stimuli. Despite the limitation in the molecular understanding in BiTE-induced immunological synapse, blinatumomab has been proved to effectively deplete the B cell lineage, considering that CD19 is widely expressed in human B cells. Therefore, blinatumomab can be considered an alternative approach to the generation of engineered CAR T cells. Compared to CAR T cells, BiTEs are versatile and cost-effective off-the-shelf therapies with the potential to specifically deplete B cells.

Starting from these ground-breaking observations, a similar approach to BiTEs technology could be a useful tool for treating autoimmune diseases. However, as they are in their current conformation (directed against CD19 receptor), the use of these compounds in autoimmune diseases would be still non-specific as they would induce general B-cell depletion and patients would still develop side-effects.

Membranous nephropathy (MN), an autoimmune disease affecting the kidneys, is one of the most common leading causes of nephrotic syndrome in adults. MN is an autoimmune disorder that is characterised, histologically, by the presence of subepithelial immune deposits containing antigens, IgG and complement components.¹³ The progressive accumulation of immune deposits induces thickening of the basement membrane (GBM) of the glomerulus, the filtering unit of the kidney, causing massive proteinuria in the urine.¹³ In about one-third of cases, persistent glomerular damage and proteinuria lead to end-stage renal disease requiring renal replacement therapy, such as dialysis or transplantation. In patients with MN, alkylating agents (cyclophosphamide or chlorambucil) alone or in combination with steroids are the first line therapies, which achieve remission of nephrotic syndrome more effectively than conservative treatment or steroids alone. However, these treatments can cause myelotoxicity, infections, and cancer. Calcineurin inhibitors can improve proteinuria as well, but are nephrotoxic. In addition, most patients relapse after treatment withdrawal and frequently become treatment-dependent, increasing the risk of nephrotoxicity. For this reason, novel pathogenic targets are urgently needed so that more specific and effective therapeutic interventions for MN can be developed.

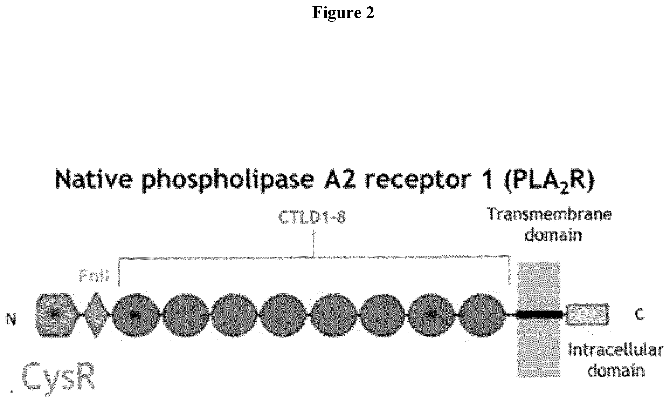

In 2009, Beck and colleagues made the landmark discovery that phospholipase A₂ receptor (PLA₂R) is the nephritogenic podocyte antigen that serves as a target autoantigen in MN.¹⁴ PLA₂R is one of four members of the mannose receptor family in mammals **(****Figure 2****).** The extracellular domain of PLA₂R comprises the N-terminal cysteine-rich (CysR) domain, a single fibronectin type-2 (FnII) domain, and eight C-type lectin-like domains (CTLD1-7).¹⁵ By probing the truncated PLA₂R domains, Kao and colleagues successfully determined that the dominant epitope for autoantibody binding is located in the N-terminal region of the extracellular domain of PLA₂R, specifically a region encompassing the CysR-FnII-CTLDI domains.¹⁶

Circulating autoantibodies against PLA₂R, predominantly of the IgG₄ subclass, were found in 70-80% of MN cases.¹³ Despite IgG₄ are considered immunologically inert and functionally monovalent due to structural differences compared to other IgG subclasses, recent evidence suggested that IgG₄ are pathogenic in primary MN.¹⁷ In line with these findings, IgG₄ anti-PLA₂R are correlated with the activity and clinical manifestations of the disease, as well as with its long-term outcomes.¹⁸

### Description of the invention

It was surprisingly found that the drawbacks associated with the use of mAbs and BiTE technologies can be overcome by means of a bi-specific autoantigen-killer T cell engager ("BiAATE") which is able to selectively target the autoreactive B cells expressing an autoantigen-specific surface immunoglobulin (sIg-BCR). With the use of BiAATE, autoreactive memory B cell and short-lived plasmablasts bearing slg recognizing the autoantigens are selectively targeted for depletion, without affecting the whole healthy B cell lineage, thereby providing a novel and safer therapeutic approach to primary MN

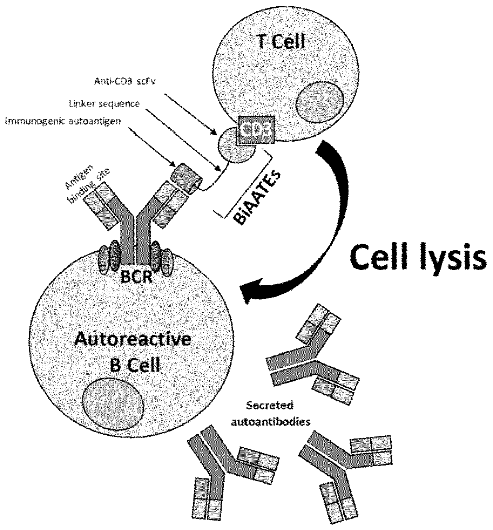

The BiAATE according to the invention is a fusion protein combining an anti-CD3 antibody and the autoantigen involved in the autoimmune disease. The anti-CD3 antibody forms the T cell engager portion of the molecule while the autoantigen forms the B cell ligand within the same molecule **(****Figure 3****).** In the case of membranous nephropathy, the autoantigen is the phospholipase A₂ receptor (PLA₂R).

In order to increase its effectiveness, the fusion protein should be able to form an immunological synapse between the T and B cells, causing the release of inflammatory cytokines, the production of cytolytic proteins and the proliferation of T cells, ultimately resulting in the lysis of the engaged CD19 B cells. Better results are obtained when the molecule size is such to enable a close proximity between the B and T cells. This can be achieved by using an antigen-binding fragment of the anti-CD3 antibody and a suitable fragment of the autoantigen which in the case of PLA₂R is located in the extracellular portion of the molecule including the N-terminus.

Accordingly, in a first aspect the invention provides a fusion protein comprising a N-terminal fragment of the phospholipase A₂ receptor (PLA₂R) extracellular domain fused to an antigen-binding fragment of an anti-CD3 antibody.
The N-terminal fragment of the PLA₂R extracellular domain includes the Cysteine-rich domain (CysR) and it may further include the single fibronectin type-2 domain (FnII), and from 1 to 7 C-type lectin-like domains (CTLD1-7). In one embodiment, the PLA₂R N-terminal fragment has an amino acid sequence of SEQ ID NO:8 (corresponding to amino acid residues 38-355 - NCBI sequence: NP_031392.3).

In a preferred embodiment, the antigenic fragment consists of the PLA2R CysR domain, as it was found that the majority of autoantibodies present in rituximab-treated patients with PLA₂R-related MN and nephrotic syndrome show a binding specificity for the CysR domain of PLA₂R. In another preferred embodiment, the CysR has an amino acid sequence of SEQ ID NO:1 (corresponding to amino acids 38-161 of NCBI sequence: NP_031392.3).

Any anti-CD3 antibody can be used in the construction of the BiAATE. Preferably the anti-CD3 antibody is selected from blinatumomab and muromonab. In a preferred embodiment, the antigen-binding fragment is scFv. The scFv from blinatumomab has an amino acid sequence which is selected from SEQ ID NOs:2 and 3, whereas the scFv from muromonab has an amino acid sequence which is selected from SEQ ID NOs:4 and 5.

In another preferred embodiment, the fusion protein comprises a linker which is interposed between the PLA₂R extracellular domain and the antigen-binding fragment of the anti-CD3 antibody. The linker may contain from 5 to 20 amino acid residues which are preferably selected from Gly and Ser.

In a preferred embodiment, the linker comprises an amino acid sequence which is selected from (i) (Gly-Gly-Gly-Gly-Ser)ₙ wherein n = 1, 2, 3 or 4 (SEQ ID NOs:9, 10, 11 and 12, respectively), and (ii) (Gly)ₘ, wherein m = 6, 7 or 8 (SEQ ID NOs:13, 14, and 15, respectively).

The fusion protein may also contain a purification tag, which is generally a polyhistidine fragment which is attached to the carboxy or amino terminal. The fusion protein carrying a polyhistidine tag can be purified using a nickel affinity column.

In a preferred embodiment, the fusion protein comprises the PLA₂R CysR domain, a linker and an anti-CD3 scFv. In another preferred embodiment, the fusion protein has an amino acid sequence of SEQ ID NO:6.

The present invention also provides a nucleic acid molecule encoding a fusion protein as herein disclosed.

The nucleic acid molecule comprises polynucleotide fragments encoding the PLA2R extracellular domain, the anti-CD3 antigen-binding fragment and, where present, the linker and the purification tag. In a preferred embodiment, the nucleic acid molecule has the sequence SEQ ID NO:7 or a sequence identical to it by at least 95%, preferably at least 97% and more preferably at least 99%.

Another aspect of the invention relates to an expression vector carrying the nucleic acid molecule as herein disclosed. The vector contains an expression cassette wherein the fusion protein encoding sequence is functionally linked to regulatory sequences suitable for the expression in prokaryotic or eukaryotic systems.

Another aspect of the invention relates to a host cell comprising an expression vector as herein disclosed. The host cell can be prokaryotic or eukaryotic, preferably it is a mammalian and more preferably a human cell.

Another aspect of the invention relates to a pharmaceutical composition comprising a fusion protein, a nucleic acid molecule or an expression vector as herein disclosed, together with physiologically compatible excipients. Suitable formulations for protein, nucleic acid and vector delivery are known to those skilled in the art. Examples can be found in the handbook Remington's Pharmaceutical Sciences, Mack Publishing Company, Easton, Pa.

Preferably the pharmaceutical composition comprising the fusion protein is in a form suitable for parenteral administration and particularly it is in the form of an injectable solution. Venous perfusion is particularly preferred. Furthermore, the fusion protein as well as the polynucleotides can be delivered by means of viral vectors, liposomes, nanoparticles or any other technology which is able to provide an adequate bioavailability and plasma concentration of the engager molecule.

The fusion protein or its encoding polynucleotides are conveniently used in the treatment of membranous nephropathy. The BiAATE can be administered as a single infusion at a dose from 50 to 1000 mg, e.g. from 50 to 100 mg, from 100 to 500 mg or from 500 to 1000mg in 250 to 500ml Sodium Chloride 0.9% *via* infusion pump. The infusion rate will not generally exceed 200 ml/hour. Mild to moderate infusion-related adverse reactions usually respond to a reduction of the infusion rate (from 25 to 50 ml/hour). Additional prophylactic treatments can be considered to avoid infusion-related adverse reactions, including intravenous methylprednisolone 100 mg in 100 ml Sodium Chloride 0.9% given 1 hour prior to BiAATE infusion, oral chlorphenamine 4 mg given 30 minutes prior to BiAATE infusion, and oral paracetamol 1 mg given 30 minutes prior to BiAATE infusion. Some patients may receive an additional infusion of the BiAATE at 6 months whether autoantibody titers do not drop after the first administration.

Since the therapeutic treatment may be affected by circulating autoantibodies which specifically bind to the fusion protein, limiting its efficiency against IgG⁺ anti-PLA₂R B cells, a pre-treatment comprising plasma apheresis and particularly double-filtration plasmapheresis (DFPP) can be performed to eliminate the majority of circulating autoantibodies before the infusion of the BiAATE. During DFPP, plasma is separated from cellular components by a plasma filter, and is then allowed to pass through a fractionator filter. Depending on the membrane cut-off, the fractionator filter retains larger molecules and returns fluid along with smaller molecules to the circulation. Thus, the selection of a membrane with an appropriate sieving coefficient for IgG allows to efficiently clear autoantibodies in patients with antibody-mediated diseases with negligible fluid losses and limited removal of albumin and coagulation factors. A similar approach recently proved effective in accelerating the depletion of circulating anti-PLA₂R antibodies in patients with severe MN

A further advantage of the invention BiAATE is the ability to target autoreactive long-live memory plasma cells. A recent study identified the existence of a unique, enriched population of IgG-expressing plasma cells lacking CD19 in human bone marrow.¹⁹ It is possible that this peculiar subset of IgG⁺/CD19⁻ plasma cells are responsible for the persistence of humoral immunity in individuals regardless of B cell depletion. Hence, their suppression by the BiAATE allows for a longer and increased therapeutic effect.

The invention will be further illustrated by the following Figures and examples.

### Description of the Figures

**Figure 1****.** Schematic representation of the available approaches to redirect the cytotoxic activity of T cells against tumor B cells by engineered chimeric antigen receptor in T cells (CAR T) or bi-specific T cell engager (BiTE) blinatumomab.
**Figure 2****.** A schematic representation of the domain structures of the native M-type phospholipase A2 receptor (PLA₂R), a large transmembrane glycoprotein with short cytoplasmic tails. The extracellular domain comprises N-terminal cysteine-rich (CysR) domain, a single fibronectin type-2 (FnII) domain, and eight C-type lectin-like domains (CTLD1-7). The most N-terminal region of the extracellular domain (CysR-FnII-CTLDI) contains the known humoral epitopes, indicated by asterisks.
**Figure 3****.** A schematic representation of the proposed mechanism of action of bi-specific autoantigen-killer T cell engagers (BiAATEs) that redirect the lytic activity of T cells against the small subset of autoreactive B cells involved in the development of autoimmune diseases.

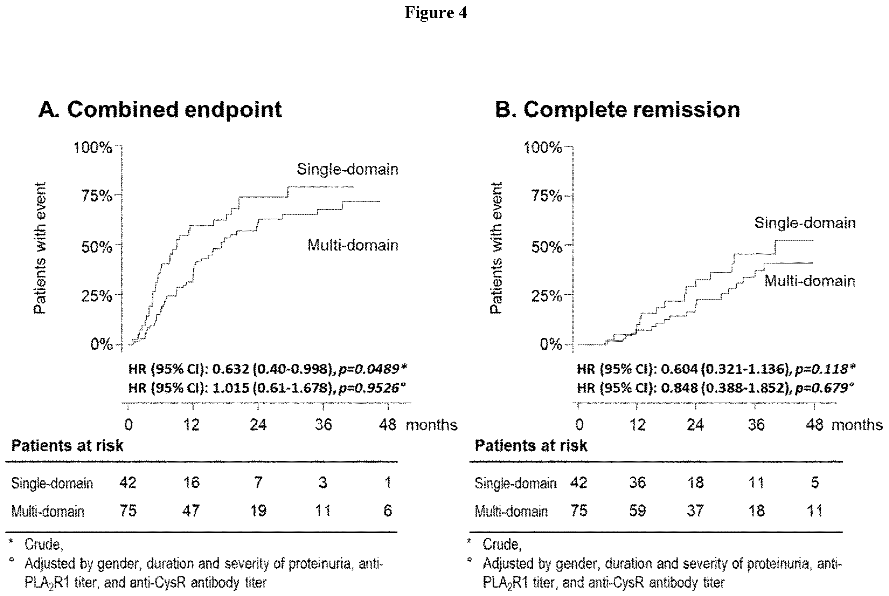

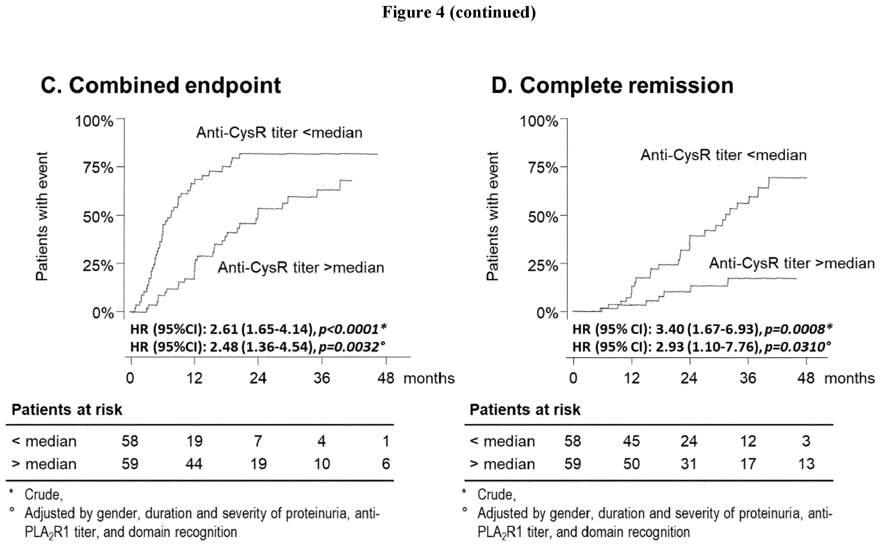

**Figure 4****. (A)** Patients with Cystein Rich (CysR)-only recognition were more likely to progress to the combined endpoint of complete and partial remission compared to those with multi-domain recognition (p=0.0489), but the predictive value of domain recognition was lost after adjustment for duration and severity of proteinuria, gender, anti-PLA₂R1 and anti-CysR titers. **(B)** Progression to complete remission was not significantly different between patients with CysR-restricted and multi-domain recognition at univariable and multivariable analyses. **(C** and **D)** Patients with anti-CysR titer below the median were more likely to progress to the combined endpoint **(C)** or to complete remission **(D)** compared to those with anti-CysR titer above the median (p<0.0001 and p=0.0008, respectively), even after adjusting for gender, duration and severity of proteinuria, domain recognition and anti-PLA2R1 titer (p=0.0032 and p=0.0310, respectively).

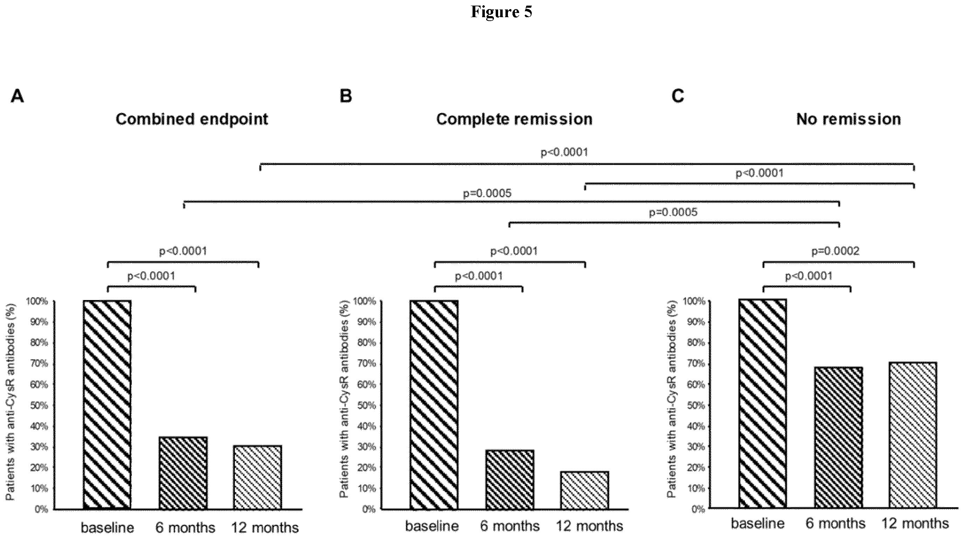

**Figure 5****. (A-C)** Data are shown in the study population considered in patients progressing to the combined endpoint of partial or complete remission **(A),** to complete remission considered as a single outcome **(B)** and patients with no remission **(C).** The prevalence of antibody against the Cystein Rich (CysR) significantly declined in all considered subgroups at 6 and 12 months after rituximab treatment *versus* baseline **(A-C).** The decline in anti-CysR antibodies prevalence at 6 and 12 months after rituximab administration was significantly larger in patients who progressed to the combined endpoint or to complete remission compared to those without remission **(A-C).**

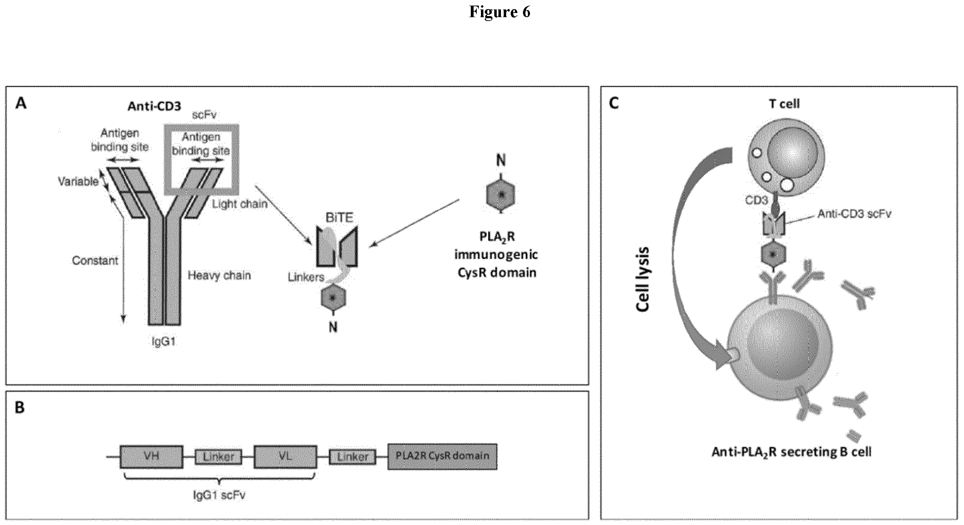

**Figure 6****. (A-B)** Schematic representation for the development of bi-specific autoantigen-T cell engagers (BiAATEs), which are hybrid synthetic molecules that express the single-chain variable fragmentanti-CD3 on one side and the CysR antigen on the other side, linked by a small flexible linker. **(C)** BiAATEs have the potential to selectively redirect T cells against surface immunoglobulin (sIg)⁺ anti-M-type phospholipase A2 receptor (PLA₂R) B cells that produce the disease-causing antibodies.

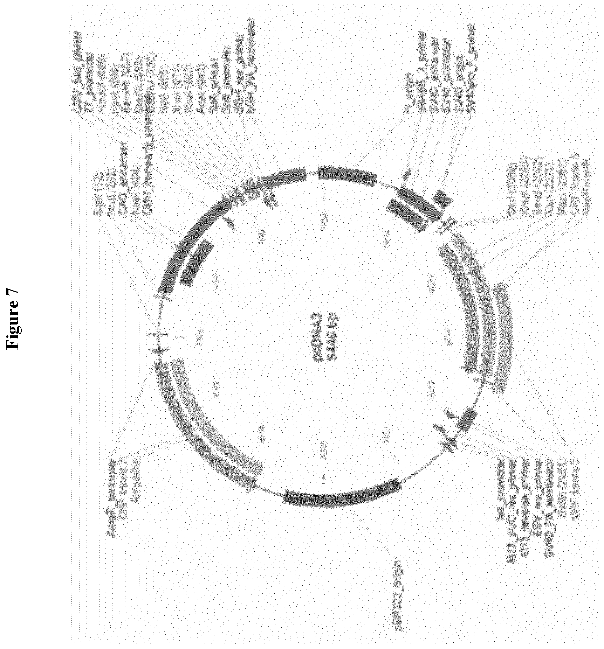

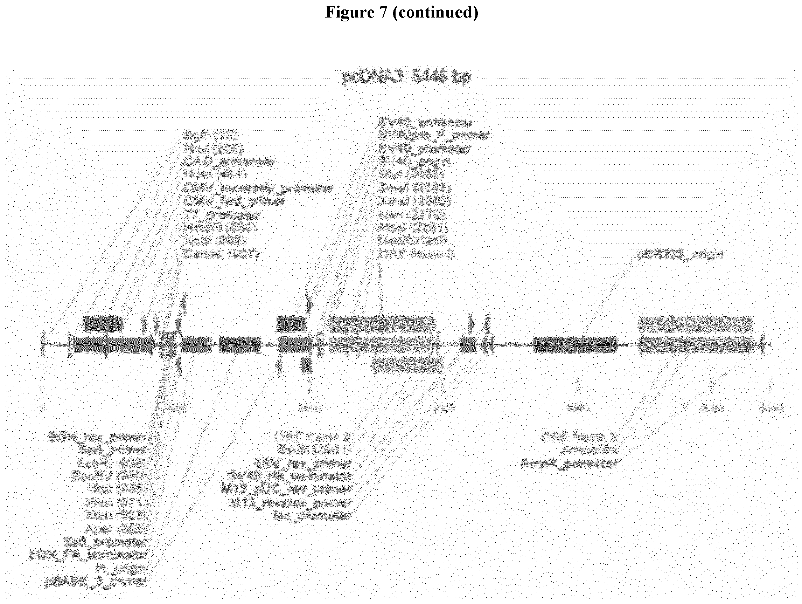

**Figure 7****.** Schematic representation of the circular and linear map of the pcDNA3 expression vector used for transfection of bi-specific autoantigen-T cell engagers (BiAATEs) DNA in HEK293 cells.

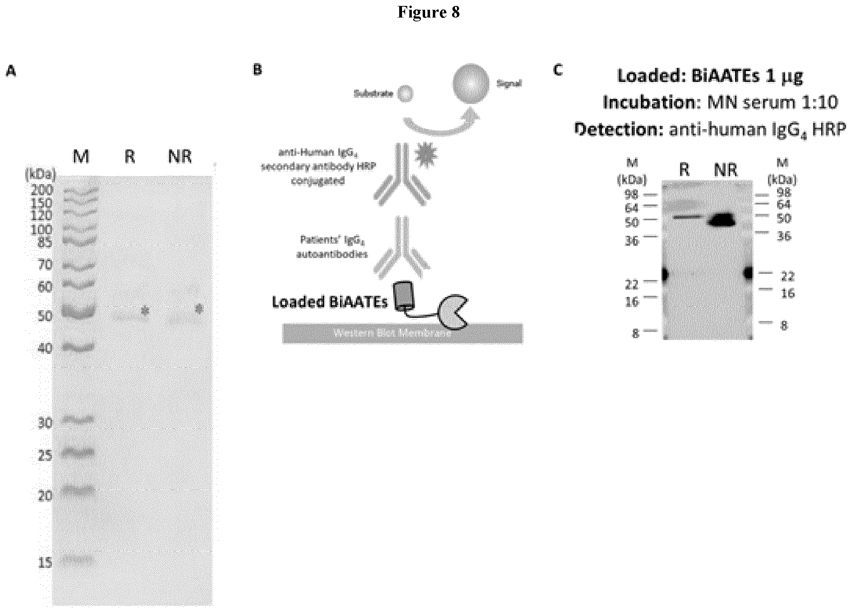

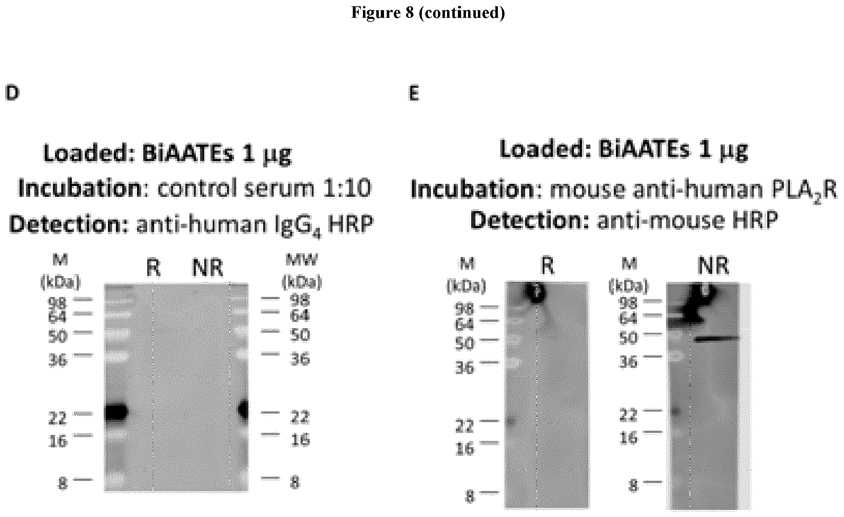

**Figure 8****. (A)** Representative western blot analysis of bi-specific autoantigen-T cell engagers (BiAATEs) in reducing **(R)** and non-reducing (NR) condition, that exhibited a molecular weight of approximatively 50 kilo Dalton (kDa), based on the markers (M) in the first lane. **(B)** Schematic representation of western blot performed to test the immunogenic properties of the CysR antigen included in the BiAATEs. **(C-E)** Representative western blot analysis of BiAATEs in R and NR condition incubated with **(C)** the serum from a membranous nephropathy (MN) patient, **(D)** the serum from a healthy volunteer (control), and **(E)** a commercially available M-type phospholipase A2 receptor (PLA₂R) antibody.

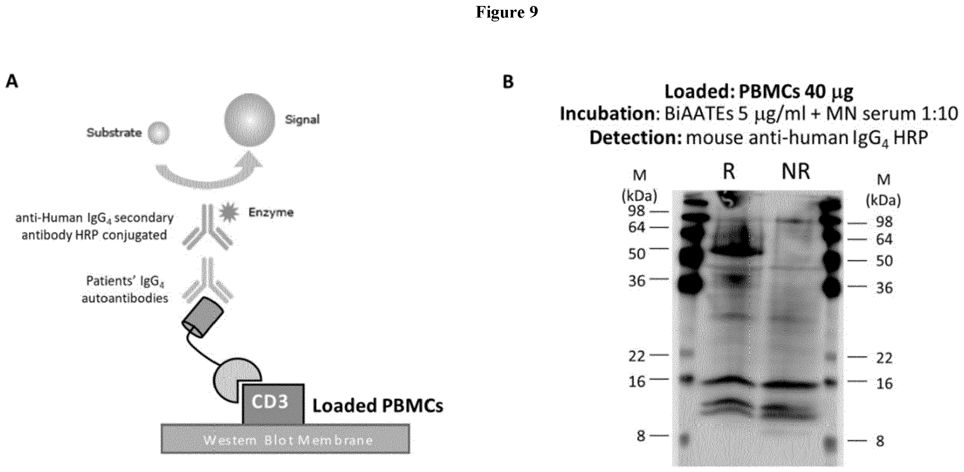

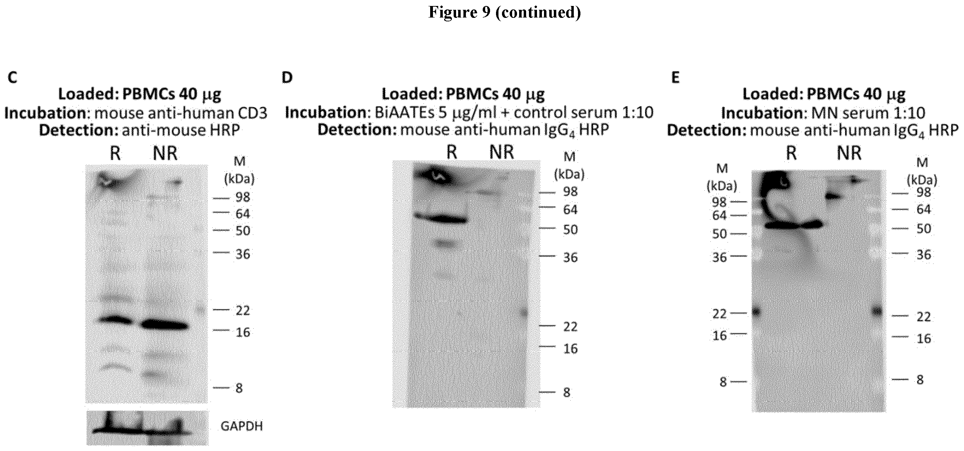

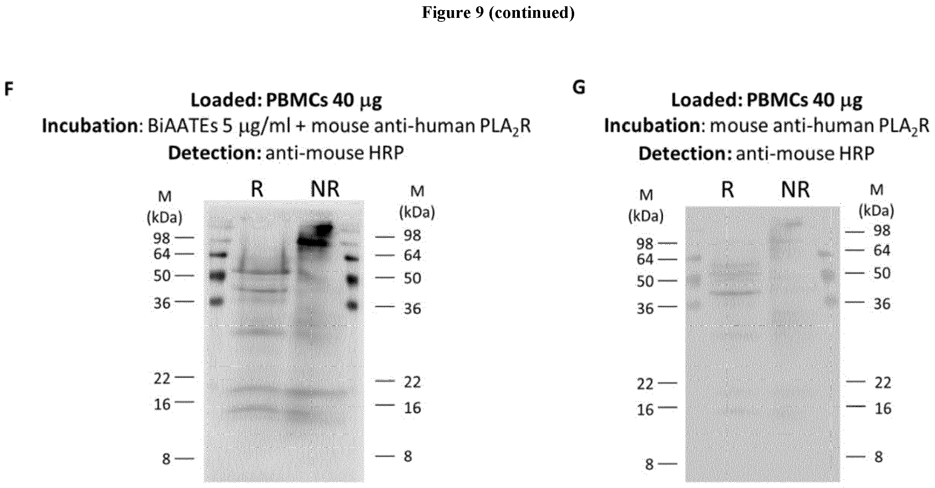

**Figure 9****. (A)** Schematic representation of western blot performed to test the ability of immunogenic properties of bi-specific autoantigen-T cell engagers (BiAATEs) to simultaneously bind CD3 in T cells and the anti-Cystein Rich (CysR) in the sera of membranous nephropathy (MN) patients. **(B)** Representative western blot analysis of total extracts of peripheral blood mononuclear cells (PBMCs) harvested from a healthy donor in reducing (R) and non-reducing (NR) condition exposed to BiAATEs followed by incubation with the serum from a MN patient. Markers (M) are expressed in kilo Dalton (kDa). **(C)** Representative western blot analysis of CD3 expression in total extracts of PBMCs. Glyceraldehyde 3-phosphate dehydrogenase (GAPDH) was use as a sample loading control. **(D)** Representative western blot analysis of total extracts of PBMCs exposed to BiAATEs followed by incubation with the serum from a healthy volunteer (control). **(E-F)** Representative western blot analysis of total extracts of PBMCs exposed to **(E)** MN and **(F)** control serum the serum without the incubation with BiAATEs. **(G)** Representative western blot analysis M-type phospholipase A2 receptor (PLA₂R) in total extracts of PBMCs.

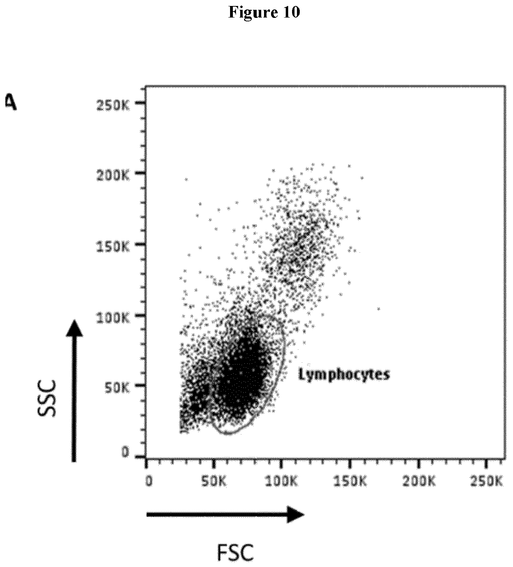

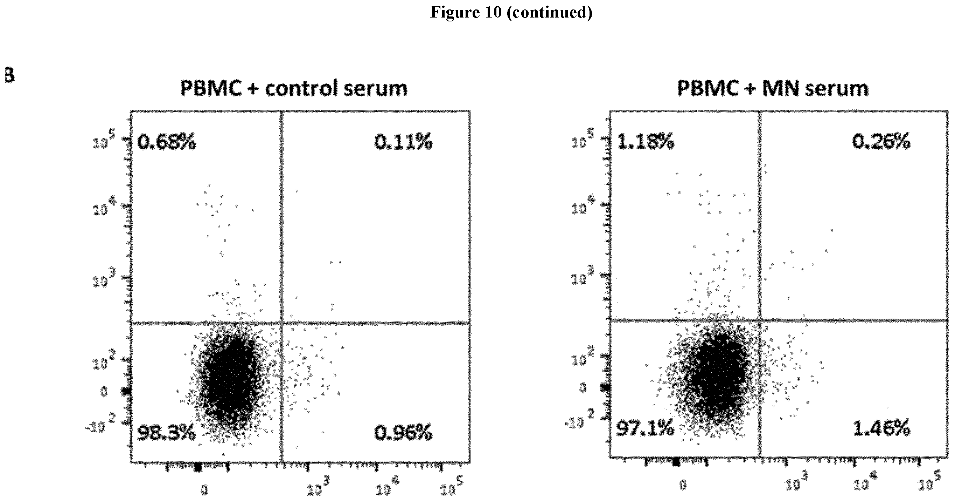

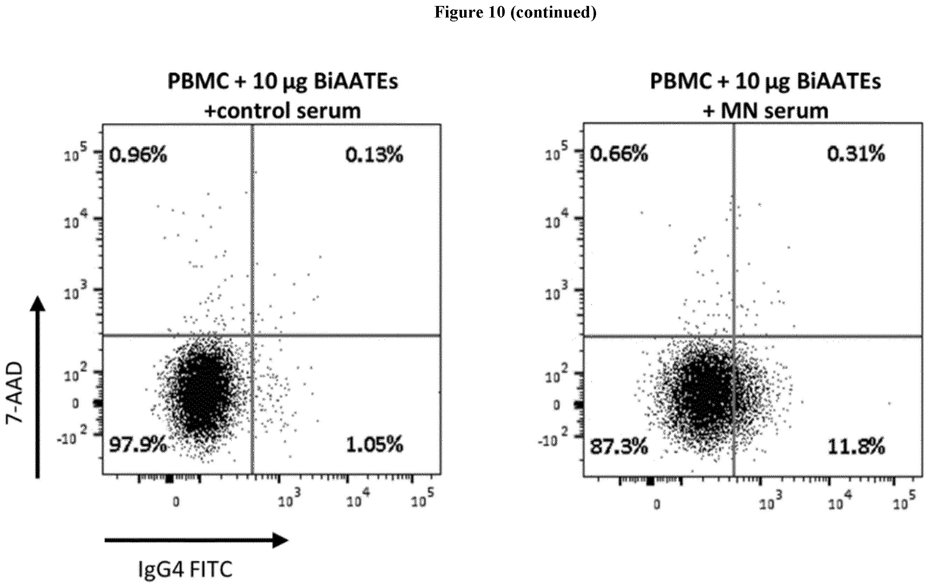

**Figure 10****. (A)** Singlet lymphocytes within peripheral blood mononuclear cell (PBMC) population were gated based on their morphologic profile. **(B)** PBMC were incubated without (upper panels) or with 10 µg bi-specific autoantigen-T cell engagers (BiAATEs, lower panels) in the presence of control serum or membranous nephropathy (MN) serum. At the end of the incubation, PBMCs were labelled with 7-AAD to identify live 7AAD negative cells and incubated with anti-human IgG₄ FITC mAb. Representative dot plots show binding of anti-IgG⁴ antibody in live PBMC incubated with 10 µg BiAATEs and serum from a MN patient.

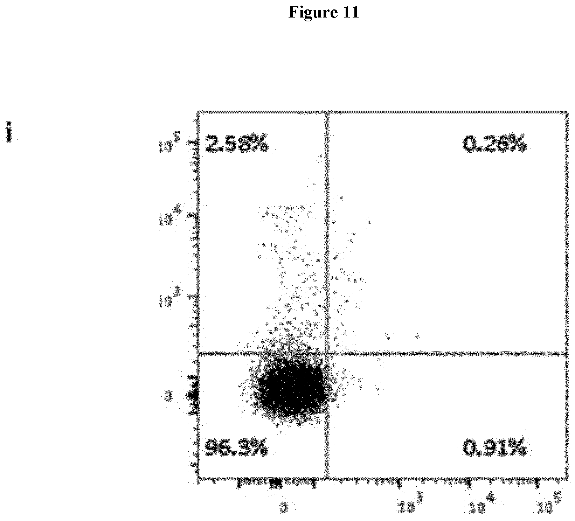

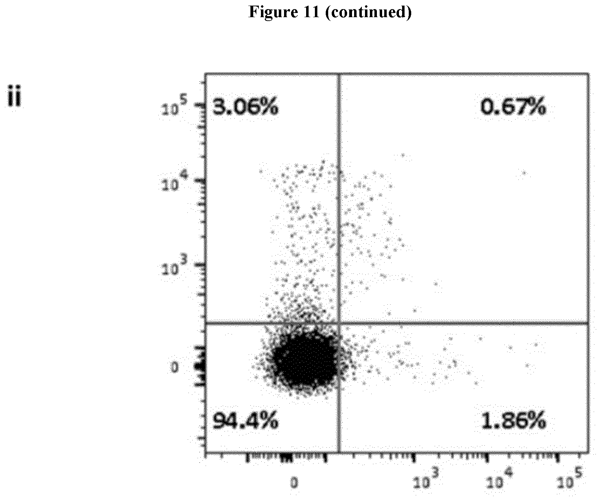

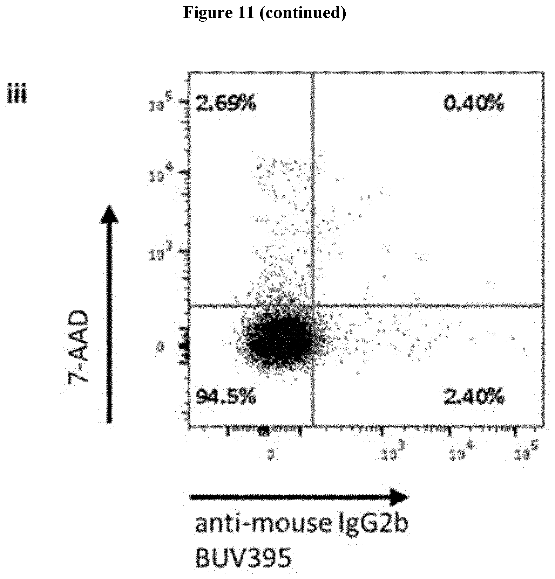

**Figure 11****.** Representative dot plots of M-type phospholipase A2 receptor (PLA₂R) binding to live peripheral blood mononuclear cells (PBMCs) after incubation with (i) PBS alone, (ii) anti-PLA₂R antibody, or (iii) 10 µg bi-specific autoantigen-T cell engagers (BiAATEs) + anti-PLA₂R mouse antibody (mAb). All samples are then incubated with the BUV395-conjugated anti-mouse IgG2b secondary antibody. Representative dot plots suggest lack of anti-PLA₂R mAb binding to PBMC exposed to BiAATEs.

### Example 1

### Characterization of PLA2R epitopes

In order to characterize the PLA₂R epitopes recognized by autoantibodies in a MN patient cohort, we assessed the specificity of the anti-PLA₂R antibodies in 117 consecutive, rituximab-treated patients with PLA₂R-related MN and nephrotic syndrome.

To this aim, series of human PLA₂R deletion mutants were generated by PCR and cloned into the pLPCX expression vector (Clontech), as previously described.²⁰ In details, deletion mutants were generated using the Phusion Site-Directed Mutagenesis Kit (Thermo Fisher Scientific). All mutants comprised the PLA₂R signal peptide (Met-1 to Ala-20) and the N-terminal linker sequence (Ala-21 to Trp-35) followed by the PLA₂R sequence with the following domains deleted at the linker regions: deletion of the N-terminal domain CysR (ΔC, deletion from Gln-36 to Asp-165), CysR and FNII domains (ΔF, Gln-36 to Thr-223), CysR to CTLD1 domains (Δ1, Gln-36 to Tyr-357), CysR to CTLD2 domains (Δ2, Gln-36 to Ala-504), CysR to CTLD3 domains (Δ3, Gln-36 to Pro-660), CysR to CTLD4 domains (Δ4, Gln-36 to Lys-805), CysR to CTLD5 domains (Δ5, Gln-36 to Lys-947), CysR to CTLD6 domains (Δ6, Gln-36 to His-1105), and CysR to CTLD7 domains (Δ7, Gln-36 to Pro-1235). All mutants were generated from a C-terminal HA-tagged (HA:YPYDVPDYA) version of the human full-length PLA₂R cDNA cloned into the pGEMTeasy vector (Promega). After full sequencing, all deletion constructs were subcloned into the pLPCX retroviral vector and transfected into human embryonic kidney cells (HEK-293, ATCC ^{®} CRL-1573^{™} Homo sapiens) using a Ca/PO4 transfection kit (InvitroGen) or Exgen (Biomol GmbH).

Soluble PLA₂R mutants were generated by PCR as above and cloned into the pcDNA3.1Z-expression vector (Life Technologies). They all comprised the PLA₂R signal peptide followed by the human PLA₂R sequence coding for the different PLA₂R domains as follows: CysR domain (CysR, from Ala-21 to Lys-164), CTLD1 domain (CTLD1 or C1, Thr-223 to Asn-359), CTLD2 to CTLD8 (C2C8, 357-Tyr to 1397-Ser), CTLD6 to CTLD8 (C6C8, 947-Lys to 1397-Ser), CTLD6 to CTLD7 (C6C7, 947-Lys to 1246-Leu), and CTLD7 to CTLD8 (C7C8, Glu-1097-1397-Ser), CTLD6 (C6, 947-Lys to 1114-Pro), CTLD7 (C7, Glu-1097-1246-Leu) and CTLD8 (C8, Pro-1235-1397-Ser). All soluble mutants were C-terminally HA-tagged. The CysR-FNII-CTLD1 domains were produced and cleaved with thrombin (Sigma-Aldrich) as described by Kao *et al*,¹⁶ except that the domains were C-terminally HA-tagged. The human codon-optimized cDNA coding for soluble CTLD1 (Thr-223 to His-377) was synthesized (Genecust, Dudelange, Luxembourg) and added in frame after the signal peptide of the human secreted phospholipase A2 PLA2G2A gene followed by a 3xFlag tag sequence. It included a C-terminal HA-tag. All mutants were expressed in HEK293 cells as described above and cell medium containing the expressed proteins were collected. When proteins were expressed at low levels, medium was precipitated with trichloroacetic acid using standard procedure or affinity-purified with anti-HA beads.

DsbC-His-HA-PLA₂R fusion proteins were produced in *E. coli* essentially as described previously.²⁰ PLA₂R domains with optimized codons for *E*. *coli* expression were added in frame with the leaderless DsbC open reading frame followed by His and HA tags for purification and detection, respectively. Sequences of inserted PLA₂R domains were as follows: CysR, Ala-26 to Lys-164; CTLD1, Thr-223 to Asn-359; CTLD7, Thr-1102 to Glu-1237; CTLD6-7, 947-Lys to Glu-1237. The four SNPs variants of CTLD1 were also designed:22 SNP1, Val-292 and Asp-300; SNP2, Met-292/Asp-300; SNP3, Met-292/His-300; and SNP4, Val-292/His-300.

Expression was confirmed by Western blot from transiently transfected HEK293 cell lysates using anti-HA antibodies, as previously described.²⁰ Briefly, the different deletion mutants and soluble forms of PLA₂R were analyzed by SDS-PAGE under non-reducing conditions. Total proteins (10-50 µg/well to adjust for the different expression level of each deletion mutant) and cell medium (for transfected soluble forms) were run on 4%-15% precast TGX SDS-PAGE gels (Bio-Rad) and transferred to methanol-soaked polyvinylidene difluoride membranes (Bio-Rad) under semi-dry conditions using Trans-blot Turbo (Bio-Rad) at 25 V constant for 12 min. For the soluble forms difficult to detect by Western blot, 0.05% SDS was added to the transfer buffer. Membranes were blocked overnight at 4°C in 5% milk with PBS-Tween (PBS-T) 0.05% and then incubated with primary and secondary antibodies for 2 h at room temperature. Primary antibodies were diluted with 0.5% dry milk in PBS-T. Membranes were prepared in multiple replicates and probed with a mouse monoclonal anti-HA antibody (Sigma-Aldrich) at 1:5000 to validate expression or with the 50 different MN sera to screen for the epitope profile at a working dilution of 1:25-1:500, depending on anti-PLA2R1 titers. The secondary antibody for anti-HA was a goat anti-mouse IgG (Southern Biotech #1030-05) diluted 1:20,000 in PBS-T. IgG secondary antibody for MN sera was horse radish peroxidase-conjugated mouse anti-human IgG₄ (Southern Biotech #9200-05) diluted 1:30,000 in PBS-T. A panel of subclass specific anti-IgGs from Southern Biotech and two different anti-total IgGs (Southern Biotech and Santa Cruz Biotechnology) were used for anti-PLA₂R IgG subclass analysis. Purified IgGs for validation of IgG subclass specificity were from Fitzgerald. Membranes were washed three times for 5 min in PBS-T after incubation with primary and secondary antibodies. The detection of protein bands was performed with a chemiluminescent substrate (EMD Millipore) and a Fuji LAS3000 imager.

To assess the specificity of autoantibodies present in MN sera, an enzyme-linked immunosorbent assay (ELISA) was developed.²⁰ Briefly, plates were coated with anti-HA antibody (Sigma-Aldrich) diluted at 1:5000 in 20 mM Tris pH 8.0 (100 µl/well) at 4°C/overnight. Plates were then blocked for 2 h with SeramunBlock (Seramun Diagnostica). Cell medium from HEK293 cells transfected with the soluble forms of PLA₂R (10-100 µl/well depending on protein expression) or purified *E. coli* DsbC-HA-CTLD1 fusion protein (50 ng/well) were then added and incubated for 1 h. Plates were washed and patients' sera diluted at 1:100 (or higher as needed) in PBS/0.1% dry milk were added in duplicate (100 µl per well) to the ELISA plates, which also contained a serial dilution of a standard MN serum and a quality control calibrator (between plates). After 2 h incubation at room temperature on a plate shaker, plates were washed four times with PBS/0.02% Tween 20. Anti-human IgG₄-horse radish peroxidase conjugate (Southern Biotech #9200-05) diluted 1:7500 in SeramunStab ST plus was added (100 µl per well; Seramun Diagnostica) and incubated for 1 h at room temperature on a plate shaker. Subclass analyses were performed with specific anti-IgGs as indicated above. After four washes, tetramethylbenzidine was added, and the reactions were developed for 15 min and then stopped with HCl 1.2N. The plates were read at 450 nm. Twenty sera from healthy donors were used to define the normal range, using mean+3SD. The cut-off was optimized by receiver operating characteristics curve analysis. A highly positive index patient serum was used in each plate to generate a standard curve and a negative control.

An ELISA Index value for each antigen was obtained for patients or normal subjects as follows: (mean test result-mean domain negative control)/(mean domain positive control-mean domain negative control)×domain correction factor×100. The domain correction factor was determined for each domain as the mean of all the positive controls for that domain on all plates minus the mean of the negative controls, divided by the cut-off for that domain assay as previously described.²⁰

We found that antibodies against the CysR domain of PLA₂R were present in all the 117 consecutive, rituximab-treated patients with PLA₂R-related MN and nephrotic syndrome **(****Figure 4** and **5****).** In this cohort, lower anti-CysR titer at the time of rituximab administration predicted higher probability of remission along with shorter duration of proteinuria. Additionally, better long-term response to rituximab is predicted by anti-CysR antibody depletion or titre reduction early post-treatment **(****Figure 4** and **5****).** Based on these data, the generation of BiAATEs containing only the CysR domain should be applicable to the entire MN patient population. Considering the small size of the CysR domain (109 amino acids), the BiAATE molecule containing it represents the best option to reduce the T and B cell intermembrane distance and enhance BiAATE efficacy.

### Example 2

### BiAATE generation

Based on the above findings, a BiAATE exhibiting an anti-CD3 antibody on one side of the molecule and the CysR autoantigen on the other side of the molecule was developed **(****Figure 6****).** The full DNA sequences comprising the sequence of the human CysR domain (38-161), a small flexible (GGGGS)₃ linker, and the scFv of anti-CD3 antibody based on blinatumomab was synthesized as shown in **Figure 7A**. The DNA was then cloned into a pcDNA3-based expression vector (V79020, Invitrogen; **Figure 7B**) using standard molecular cloning techniques with optimized codons for transfection in HEK293 cells. HEK293 cells transiently transfected with this vector using Lipofectamine, ultimately expressing the fusion peptide composed by the CysR domain, a polypeptide (G4S)3 linker, and the single-chain variable fragment (scFv) anti-CD3 antibody **(****Figure 7C****).** The BiAATE also included a C-terminal 6His-tag which allows its purification using a nickel affinity column. The expression period was 5 days. Nickel columns (Bio-Rad) were used for immobilized metal affinity chromatography (IMAC) for the purification of the recombinant proteins with the 6His-tag. The purified protein was then subjected to ultrafiltration and 0.2 µm sterile filtration to get the bulk of high purity. The Limulus Amebocyte Lysate (LAL) test was used for detecting bacterial toxins the final high purity product. The protein concentration was calculated with nanodrop with the extinction coefficient of 2.177, identifying a final protein concentration of 0.02 mg/ml.

### Example 3

### In vitro testing of BiAATE

Western blot analysis was performed in order to validate the generated BiAATE. Briefly, 1 µg of the bispecific fusion protein (50 µl) was loaded on 12% SDS-PAGE under reducing and non-reducing conditions and transferred to nitrocellulose membranes (Bio-Rad Laboratories). As shown in **Figure 8A****,** ponceau red staining (Sigma-Aldrich) revealed that the bispecific fusion protein exhibited a molecular weight of approximatively 50 kilo Dalton (kDa) basing on pre-stained molecular weight standards (SeeBlue^{™} Plus2 Pre-Stained Protein Standard, Invitrogen). The molecular weight of the BiAATE is not different from that of blinatumomab (54 kDa), suggesting that the bispecific fusion protein has a steric bulk comparable to blinatumomab that may favour the immunological synapse between T and B cells.

We then tested the autoantigen-binding capability of the CysR antigen included in BiAATEs as schematically depicted in **Figure 8B****.** To this aim, 1 µg of the bispecific fusion protein (50 µl) was loaded on 15% SDS-PAGE under reducing and non-reducing conditions and transferred to nitrocellulose membranes (Bio-Rad Laboratories). After blocking with 5% bovine serum albumin (BSA) in Tris-buffered saline (TBS) supplemented with 0.1% Tween-20, membranes were incubated with serum from a MN patient (1:10), serum from a healthy control (1:10), or with a commercially available mouse anti-human PLA₂R antibody (1 µg/ml; abcam, ab211490). The detection was performed using a mouse anti-human IgG₄ HRP-conjugated secondary antibody for samples incubated with patient's or control' sera (Invitrogen, A-10654) or with a goat anti-mouse HRP-conjugated secondary antibody for samples exposed to a commercially available anti-PLA₂R antibody (Invitrogen, 31430). The signals were visualized on an Odyssey^{®}FC Imaging System (LiCor) and bands were quantified by using the Image Studio Lite 5.0 (LiCor) software.

As shown in **Figure 8C****,** the CysR domain included in the bispecific fusion protein was recognized by pathogenic IgG₄ autoantibodies from patients' serum, particularly in non-reducing condition. No reactivity was found when the bispecific fusion protein was incubated with healthy control' serum **(****Figure 8D****).** The CysR domain included in the bispecific fusion protein was recognized also by the commercially available antibody raised against the N-terminal domain of PLA₂R **(****Figure 8E****),** unless to a lesser extent compared to pathogenic IgG₄ autoantibodies from patients' serum. Also in this setting, the CysR domain included in the bispecific fusion was recognized only in non-reducing condition **(****Figure 8E****).**

We then performed western blot experiments to evaluate the potential of the generated BiAATE to simultaneously bind CD3 and anti-PLA₂R autoantibodies as schematically depicted in **Figure 9A****.** To this aim, peripheral blood mononuclear cells (PBMCs), which contains T lymphocytes were isolated from 50 ml of fresh EDTA-treated peripheral blood obtained from healthy donors by using Ficoll-Paque density centrifugation (Histopaque, 1077 from Sigma Aldrich) following standard procedures. Isolated PBMCs were counted and cell viability was assessed by trypan blue staining (Invitrogen).

For western blot analysis, 10x10⁶ PBMCs were homogenized in 300 µl of CelLytic M (Sigma-Aldrich, C2978) supplemented with protease inhibitor cocktail (Sigma-Aldrich, P8340) containing 104 mM AEBSF at, 80 µM Aprotinin, 4 mM Bestatin, 1.4 mM E-64, 2 mM Leupeptin and 1.5 mM Pepstatin A. After sonication, cell disruption was completed by using a blunt-ended needle and a syringe and the sample lysates were then centrifuged at 16,000xg for 10 minutes at 4°C to remove detergent-insoluble material. Total protein concentration was determined using DC^{™} assay (Bio-Rad Laboratories, 5000112).

Equal amounts of total PBMC extracts (40 µg) were loaded on 15% SDS-PAGE under reducing and non-reducing conditions and transferred to nitrocellulose membranes (Bio-Rad Laboratories). After blocking with 5% bovine serum albumin (BSA) in Tris-buffered saline (TBS) supplemented with 0.1% Tween-20, membranes were incubated with the bispecific fusion protein (5 µg/ml), followed by serum from a MN patient (1:10), serum from a healthy control (1:10), or commercially available anti-human PLA₂R antibody (1 µg/ml; abcam, ab211490). As a control, a commercially available mouse anti-human CD3 antibody (Biocare Medical, PA0553 clone LN10) was used. The detection was performed using a mouse anti-human IgG₄ HRP-conjugated secondary antibody for autoantibodies in patient's or control' sera (Invitrogen, A-10654) or a goat anti-mouse HRP-conjugated secondary antibody for commercially available anti-PLA₂R antibody and anti-CD3 (Invitrogen, 31430). The signals were visualized on an Odyssey^{®}FC Imaging System (LiCor) and bands were quantified by using the Image Studio Lite 5.0 (LiCor) software.

Our results show that the bispecific fusion protein is able to bind simultaneously CD3 in PBMC extracts and the pathogenic IgG₄ autoantibodies in MN patient serum as documented by a positive signal at about 16 kDa **(****Figure 9B****),** corresponding to CD3 on T lymphocytes. The binding specificity of the bispecific fusion protein to CD3 was confirmed using a commercially available mouse anti-human CD3 antibody **(****Figure 9C****).** No reactivity was found when PBMC extracts were incubated with the bispecific fusion followed by healthy control serum **(****Figure 9D****)** or when PMBC extract were exposed to serum from a MN patient without BiAATE **(****Figure 9E****).** When total PBMC extracts were incubated with the bispecific fusion protein (5 µg/ml), followed by commercially available anti-human PLA₂R antibody (1 µg/ml) a very weak reactivity against CD3 was found **(****Figure 9F****),** possibly suggesting that anti-CysR IgG₄ in patients' sera have more affinity for the CysR domain included in BiAATEs compared to a commercially available anti-PLA₂R antibody generated from the recombinant fragment corresponding to the extracellular N-terminal sequence (corresponding to human PLA₂R aa 20-663), containing the CysR-FibII-CTLD1-CTLD2-CTLD3 domain. No reactivity against CD3 was found when total PBMC extracts were incubated with a commercially available anti-human PLA₂R antibody (1 µg/ml) alone, used as a negative control **(****Figure 9G****).**

### Example 4

### Ex-vivo testing of BiAATEs

In order to evaluate *ex vivo* the potential of the bispecific fusion protein to bind simultaneously CD3 on T cells and the pathogenic IgG₄ autoantibodies, 1x10⁶ live PBMCs were incubated with bispecific fusion protein (10 µg/ml) followed by healthy control' serum (1:10) or serum from a MN patient (1:10). The detection was performed by FACS (BD Fortessa X20) analysis using a mouse anti-human IgG₄ FITC (Sigma Aldrich, F9890) secondary antibody in the presence of the vitality dye 7AAD (BD).

Singlet lymphocytes within PBMCs (1x10⁶) were gated based on their morphologic profile **(****Figure 10A****)** and plotted for the binding of FITC anti-IgG4 mAb and 7-AAD (to identify live 7AAD negative cells) after incubation without (Figure **10B****,** upper panels) or with 10 µg BiAATEs (Figure **10B****,** lower panels) in the presence of control serum or MN serum. The BiAATE did not induce PBMC death at this concentration. As shown in the representative dot plots, the BiAATE was able to simultaneously bind CD3 in PBMCs and the pathogenic IgG₄ autoantibodies in MN patient' serum as shown by a 10-fold increase in the positivity for IgG₄ FITC from the 1.05% of PBMCs incubated with the BiAATE and control' serum to the 11.8% of PBMCs incubated with the BiAATE and MN patient' serum (Figure **10B****,** lower panels).

Live PBMCs were also incubated with bispecific fusion protein (10 µg/ml) followed by commercially available anti-human PLA₂R mouse antibody (1 µg/ml; abcam, ab211490). The detection was performed by FACS analysis using a BUV395 rat anti-Mouse IgG2b (BD Biosciences, 743180). As shown in **Figure 11****,** when PBMCs were incubated with the BiAATE followed by commercially available anti-human PLA₂R antibody no positivity was found, suggesting that anti-CysR IgG₄ in patients' sera are more affine to the CysR domain included in BiAATEs compared to a commercially available anti-PLA₂R antibody generated from the recombinant fragment corresponding to the extracellular N-terminal sequence (corresponding to human PLA₂R aa 20-663), containing the CysR-FibII-CTLD1-CTLD2-CTLD3 domain, confirming WB results.

Overall, these data indicate that the BiAATE molecule binds CD3 expressed by T lymphocytes and recognizes the BCR of autoreactive B Cells, as documented by the ability of the CysR domain included in the BiAATE to act as a conformational antigen that is recognized by pathogenic IgG₄ autoantibodies in MN patients' sera.

### Example 5

### Ex-vivo test of the BiAATE potential to promote the killing activity of T cells toward autoreactive B cells isolated from MN patients

A) Modifications of established flow cytometry-based antigen-bait assays are developed to identify CysR-specific B-cells in PBMC from MN patients. Fluorochrome-labelled CysR tetramers are used to screen and isolate the CysR-specific B cells from PBMCs from MN patients on the basis of their BCR specificity. To this purpose, a synthetic peptide is expressed in a HEK293 consisting in the CysR domain of PLA₂R (38-161aa, the same included in the BiAATE), an AviTag to allow C-ter BirA-mediated site-specific mono-biotinylation and an additional tag His-Tag with a furin cleavable linker for protein purification by Nickel columns (Bio-Rad) for IMAC. Fluorochrome-labelled synthetic peptide-tetramers are then generated by incubating the mono-biotinylated recombinant CysR with streptavidin-fluorochrome conjugates. Fluorochrome-labelled CysR tetramers are used to screen B-cells bearing a CysR-specific surface Ig⁺. To increase signal specificity, two different fluorochrome-bearing constructs are used, and autoreactive B cells are identified as those simultaneously labelled by both.
B) FACS-sorted anti-CysR B cells are transfected with a lentiviral construct containing the B cell lymphoma-6 (Bcl-6) and Bcl-xL proteins. By introducing these genes, transfected anti-CysR B cells are immortalized, become proliferating and maintain the expression of the surface B cell receptor (BCR), as well as IgG secretion, as previously described.
C) Immortalized anti-CysR B cells are incubated with autologous T cells purified by Pan T cell isolation kit (Miltenyi Biotec, San Diego, CA) in the presence of BiAATE. T-cell killing activity against immortalized CysR-specific B cells is monitored through flow cytometry, immunofluorescence, and time-lapse analysis and by ⁵¹Cr-release assay. Additionally, supernatants are collected after 24 hr of culture for the evaluation of the concentration of four different cytokines (IL-2, IL-10, IFN-γ and TNF-α) assessed by ELISA-based cytokine assay kits (OptEIA^{™} human cytokine set, BD Biosciences, San Jose, CA).

### Example 6

### In vivo test of the BiAATE potential to induce the death of autoreactive CysR-specific B cells in mice

A) The high human-specificity of the CD3 antibody included in the BiAATE prevents to test the safety and efficacy of BiAATEs in conventional animal models (expressing the murine CD3). To overcome this issue, commercially available mice carrying transgenic human CD3 (hCD3 mice, MI-mAbs, Marseille) are used. This strain is completely immunocompetent. These mice are immunized by giving biweekly injections of 50 µg of purified CysR antigen in Freund's Complete Adjuvant. To this aim, the CysR domain of PLA₂R (38-161aa, the same included in the BiAATE) with C-terminal 6XHis tag is generated by transient expression using either HEK 293 and protein purification is performed using Nickel affinity column. Subsequent immunizations are performed in normal saline with Incomplete Freund's adjuvant. Over the course of the 6-wk immunization schedule, each animal usually receives a total of six injections (three subcutaneous and three intraperitoneal). One week after each boost, 200 µL of serum is collected to monitor serum antibody levels (anti-PLA₂R ELISA IgG, EUROIMMUN AG, EA1254-9601G), adapted with a secondary anti-mouse antibody for the detection of anti-CysR antibodies in mice. When a sufficient antibody titer is reached, hCD3 mice are sacrificed, the spleen is harvested and splenocytes are isolated following standard procedures. Isolated splenocytes, which contain the CysR-specific B cells, are adoptively transferred into naive hCD3 mice (to avoid any possible interference of the anti-CysR antibodies formed after previous immunizations) which are given or not i.v. injection of 10 µg/kg of BiAATEs and an additional boost of CysR immunization. The ability of BiAATE to redirect *in vivo* the killing activity of T cells against anti-CysR B cells is assessed as a reduction of anti-CysR antibody titer.
B) Humanized PBMC mice are used as an additional *in vivo* model to study and evaluate the efficacy of the BiAATEs. In this setting, we took advantage of NSG-(K^{b}D^{b})^{null}(IA)^{null}, which are highly immunodeficient NOD scid gamma (NSG) mice knock out for major histocompatibility complex (MHC)I and MHCII (Jackson Laboratories). These mice allow the engraftment of human immune cell without developing acute graft versus host disease (GVHD). NSG-(K^{b}D^{b})^{null}(IA)^{null} mice are injected with immortalized anti-CysR B cells (as described in 1B) and with autologous T cells (isolated from the same MN patient) in the presence or absence of BiAATEs (10 µg/kg i.v). Following engraftment, 200 µL of serum is collected to monitor serum antibody levels (anti-PLA₂R ELISA IgG, EUROIMMUN AG, EA1254-9601G). The ability of BiAATE to redirect *in vivo* the killing activity of T cells against anti-CysR B cells is assessed as the ability of the bispecific fusion protein to reduce anti-CysR antibody titer in NSG-(K^{b}D^{b})^{null}(IA)^{null} mice. Additional mice are given CFSE-labelled immortalized anti-CysR B cells together with autologous T cells and BiAATEs as described above. In this setting, the ability of BiAATE to redirect *in vivo* the killing activity of T cells against anti-CysR B cells is assessed as the ability of the bispecific fusion protein to prevent proliferation or even the disappearance of immortalized anti-CysR B cells in-vivo.

### Bibliography

1. Walsh SJ, Rau LM. Autoimmune diseases: a leading cause of death among young and middle-aged women in the United States. Am. J. Public Health. 2000;90(9):1463-1466.
2. Autoimmune Diseases. Natl. Inst. Environ. Health Sci.*.*
3. The Cost Burden of Autoimmune Disease: The Latest Front in the War on Healthcare Spending. 14.
4. Du FH, Mills EA, Mao-Draayer Y. Next-generation anti-CD20 monoclonal antibodies in autoimmune disease treatment. Autoimmun. Highlights. 2017;8(1):12.
5. Ruggenenti P, Debiec H, Ruggiero B, et al. Anti-Phospholipase A2 Receptor Antibody Titer Predicts Post-Rituximab Outcome of Membranous Nephropathy. J. Am. Soc. Nephrol. JASN. 2015;26(10):2545-2558.
6. van den Brand JAJG, Ruggenenti P, Chianca A, et al. Safety of Rituximab Compared with Steroids and Cyclophosphamide for Idiopathic Membranous Nephropathy. J. Am. Soc. Nephrol. JASN. 2017;
7. Ruggenenti P, Fervenza FC, Remuzzi G. Treatment of membranous nephropathy: time for a paradigm shift. Nat. Rev. Nephrol. 2017;
8. Kansal R, Richardson N, Neeli I, et al. Sustained B cell depletion by CD19-targeted CAR T cells is a highly effective treatment for murine lupus. Sci. Transl. Med. 2019;11(482):.
9. Ellebrecht CT, Bhoj VG, Nace A, et al. Reengineering chimeric antigen receptor T cells for targeted therapy of autoimmune disease. Science. 2016;353(6295):179-184.
10. Demichelis-Gómez R, Perez-Samano D, Bourlon C. Bispecific Antibodies in Hematologic Malignancies: When, to Whom, and How Should Be Best Used? Curr. Oncol. Rep. 2019;21(2):17.
11. Wolf E, Hofmeister R, Kufer P, Schlereth B, Baeuerle PA. BiTEs: bispecific antibody constructs with unique anti-tumor activity. Drug Discov. Today. 2005;10(18):1237-1244.
12. Krishnamurthy A, Jimeno A. Bispecific antibodies for cancer therapy: A review. Pharmacol. Ther. 2018;185:122-134.
13. Beck LH, Salant DJ. Membranous nephropathy: from models to man. J. Clin. Invest. 2014;124(6):2307-2314.
14. Beck LH, Bonegio RGB, Lambeau G, et al. M-type phospholipase A2 receptor as target antigen in idiopathic membranous nephropathy. N. Engl. J. Med. 2009;361(1):11-21.
15. Fresquet M, Jowitt TA, Gummadova J, et al. Identification of a major epitope recognized by PLA2R autoantibodies in primary membranous nephropathy. J. Am. Soc. Nephrol. JASN. 2015;26(2):302-313.
16. Kao L, Lam V, Waldman M, Glassock RJ, Zhu Q. Identification of the immunodominant epitope region in phospholipase A2 receptor-mediating autoantibody binding in idiopathic membranous nephropathy. J. Am. Soc. Nephrol. JASN. 2015;26(2):291-301.
17. Tomas NM, Beck LH, Meyer-Schwesinger C, et al. Thrombospondin type-1 domain-containing 7A in idiopathic membranous nephropathy. N. Engl. J. Med. 2014;371(24):2277-2287.
18. Ruggenenti P, Debiec H, Ruggiero B, et al. Anti-Phospholipase A2 Receptor Antibody Titer Predicts Post-Rituximab Outcome of Membranous Nephropathy. J. Am. Soc. Nephrol. JASN. 2015;26(10):2545-2558.
19. Mei HE, Wirries I, Frolich D, et al. A unique population of IgG-expressing plasma cells lacking CD19 is enriched in human bone marrow. Blood. 2015;125(11):1739-1748.
20. Seitz-Polski B, Dolla G, Payré C, et al. Epitope Spreading of Autoantibody Response to PLA2R Associates with Poor Prognosis in Membranous Nephropathy. J. Am. Soc. Nephrol. 2016;27(5):1517-1533.

## Claims

1. A fusion protein comprising:
- an N-terminal fragment of the phospholipase A2 receptor (PLA2R) extracellular domain, and
- an antigen-binding fragment of an anti-CD3 antibody.

2. The fusion protein according to claim 1, wherein the N-terminal fragment of the PLA₂R extracellular domain comprises the Cysteine-rich domain (CysR).

3. The fusion protein according to claim 2, wherein said CysR has an amino acid sequence of SEQ ID NO:1.

4. The fusion protein according to claims 2-3, wherein said N-terminal fragment of the PLA₂R extracellular domain further comprises the single fibronectin type-2 domain (FnII) and from 1 to 7 C-type lectin-like domains (CTLD1-7).

5. The fusion protein according to claim 1, wherein said anti-CD3 antibody is blinatumomab or muromonab.

6. The fusion protein according to claim 1, wherein said antigen-binding fragment of an anti-CD3 antibody is scFv.

7. The fusion protein of claim 6, wherein said scFv is selected from SEQ ID NOs:2, 3, 4 and 5.

8. The fusion protein according to claims 1-7, further comprising a peptide linker interposed between the PLA₂R extracellular domain and the anti-CD3 antigen-binding fragment.

9. The fusion protein according to claim 7, wherein said linker contains from 5 to 20 amino acid residues.

10. The fusion protein according to claim 9, wherein said linker comprises an amino acid sequence which is selected from (i) (Gly-Gly-Gly-Gly-Ser)ₙ, wherein n = 1, 2, 3 or 4 and (ii) (Gly)ₘ, wherein m = 6, 7 or 8.

11. The fusion protein according to claims 1-10, which further comprises a purification tag.

12. The fusion protein according to claim 11, wherein said purification tag is a polyhistidine fragment.

13. A nucleic acid molecule encoding the fusion protein according to any preceding claims.

14. An expression vector comprising the nucleic acid molecule of claim 13.

15. A pharmaceutical composition comprising a fusion protein according to claims 1-12, a nucleic acid molecule according to claim 13 or an expression vector according to claim 14, together with pharmaceutically acceptable excipients.

16. A fusion protein according to claims 1-12, a nucleic acid molecule according to claim 13 or an expression vector according to claim 14 for use in the treatment of membranous nephropathy.


SEQUENCE LISTING

<110>  Kilometro Rosso

<120>  Fusion proteins and use thereof in the treatment of membranous
       nephropathy

<130>  3602EUR

<160>  15

<170>  PatentIn version 3.5

<210>  1
<211>  124
<212>  PRT
<213>  Homo sapiens

<400>  1

Lys Gly Ile Phe Val Ile Gln Ser Glu Ser Leu Lys Lys Cys Ile Gln
1               5                   10                  15

Ala Gly Lys Ser Val Leu Thr Leu Glu Asn Cys Lys Gln Ala Asn Lys
            20                  25                  30

His Met Leu Trp Lys Trp Val Ser Asn His Gly Leu Phe Asn Ile Gly
        35                  40                  45

Gly Ser Gly Cys Leu Gly Leu Asn Phe Ser Ala Pro Glu Gln Pro Leu
    50                  55                  60

Ser Leu Tyr Glu Cys Asp Ser Thr Leu Val Ser Leu Arg Trp Arg Cys
65                  70                  75                  80

Asn Arg Lys Met Ile Thr Gly Pro Leu Gln Tyr Ser Val Gln Val Ala
                85                  90                  95

His Asp Asn Thr Val Val Ala Ser Arg Lys Tyr Ile His Lys Trp Ile
            100                 105                 110

Ser Tyr Gly Ser Gly Gly Gly Asp Ile Cys Glu Tyr
        115                 120

<210>  2
<211>  243
<212>  PRT
<213>  Artificial Sequence

<220>
<223>  recombinant antibody

<400>  2

Asp Ile Lys Leu Gln Gln Ser Gly Ala Glu Leu Ala Arg Pro Gly Ala

1               5                   10                  15

Ser Val Lys Met Ser Cys Lys Thr Ser Gly Tyr Thr Phe Thr Arg Tyr
            20                  25                  30

Thr Met His Trp Val Lys Gln Arg Pro Gly Gln Gly Leu Glu Trp Ile
        35                  40                  45

Gly Tyr Ile Asn Pro Ser Arg Gly Tyr Thr Asn Tyr Asn Gln Lys Phe
    50                  55                  60

Lys Asp Lys Ala Thr Leu Thr Thr Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Tyr Tyr Asp Asp His Tyr Cys Leu Asp Tyr Trp Gly Gln Gly
            100                 105                 110

Thr Thr Leu Thr Val Ser Ser Val Glu Gly Gly Ser Gly Gly Ser Gly
        115                 120                 125

Gly Ser Gly Gly Ser Gly Gly Val Asp Asp Ile Gln Leu Thr Gln Ser
    130                 135                 140

Pro Ala Ile Met Ser Ala Ser Pro Gly Glu Lys Val Thr Met Thr Cys
145                 150                 155                 160

Arg Ala Ser Ser Ser Val Ser Tyr Met Asn Trp Tyr Gln Gln Lys Ser
                165                 170                 175

Gly Thr Ser Pro Lys Arg Trp Ile Tyr Asp Thr Ser Lys Val Ala Ser
            180                 185                 190

Gly Val Pro Tyr Arg Phe Ser Gly Ser Gly Ser Gly Thr Ser Tyr Ser
        195                 200                 205

Leu Thr Ile Ser Ser Met Glu Ala Glu Asp Ala Ala Thr Tyr Tyr Cys
    210                 215                 220

Gln Gln Trp Ser Ser Asn Pro Leu Thr Phe Gly Ala Gly Thr Lys Leu
225                 230                 235                 240

Glu Leu Lys

<210>  3
<211>  250
<212>  PRT
<213>  Artificial Sequence

<220>
<223>  recombinant antibody

<400>  3

Asp Ile Gln Leu Thr Gln Ser Pro Ala Ser Leu Ala Val Ser Leu Gly
1               5                   10                  15

Gln Arg Ala Thr Ile Ser Cys Lys Ala Ser Gln Ser Val Asp Tyr Asp
            20                  25                  30

Gly Asp Ser Tyr Leu Asn Trp Tyr Gln Gln Ile Pro Gly Gln Pro Pro
        35                  40                  45

Lys Leu Leu Ile Tyr Asp Ala Ser Asn Leu Val Ser Gly Ile Pro Pro
    50                  55                  60

Arg Phe Ser Gly Ser Gly Ser Gly Thr Asp Phe Thr Leu Asn Ile His
65                  70                  75                  80

Pro Val Glu Lys Val Asp Ala Ala Thr Tyr His Cys Gln Gln Ser Thr
                85                  90                  95

Glu Asp Pro Trp Thr Phe Gly Gly Gly Thr Lys Leu Glu Ile Lys Gly
            100                 105                 110

Gly Gly Gly Ser Gly Gly Gly Gly Ser Gly Gly Gly Gly Ser Gln Val
        115                 120                 125

Gln Leu Gln Gln Ser Gly Ala Glu Leu Val Arg Pro Gly Ser Ser Val
    130                 135                 140

Lys Ile Ser Cys Lys Ala Ser Gly Tyr Ala Phe Ser Ser Tyr Trp Met
145                 150                 155                 160

Asn Trp Val Lys Gln Arg Pro Gly Gln Gly Leu Glu Trp Ile Gly Gln
                165                 170                 175

Ile Trp Pro Gly Asp Gly Asp Thr Asn Tyr Asn Gly Lys Phe Lys Gly
            180                 185                 190

Lys Ala Thr Leu Thr Ala Asp Glu Ser Ser Ser Thr Ala Tyr Met Gln
        195                 200                 205

Leu Ser Ser Leu Ala Ser Glu Asp Ser Ala Val Tyr Phe Cys Ala Arg

210                 215                 220

Arg Glu Thr Thr Thr Val Gly Arg Tyr Tyr Tyr Ala Met Asp Tyr Trp
225                 230                 235                 240

Gly Gln Gly Thr Thr Val Thr Val Ser Ser
                245                 250

<210>  4
<211>  260
<212>  PRT
<213>  Artificial Sequence

<220>
<223>  recombinant antibody

<400>  4

Gln Val Gln Leu Gln Gln Ser Gly Ala Glu Leu Val Arg Pro Gly Ser
1               5                   10                  15

Ser Val Lys Ile Ser Cys Lys Ala Ser Gly Tyr Ala Phe Ser Ser Tyr
            20                  25                  30

Trp Met Asn Trp Val Lys Gln Arg Pro Gly Gln Gly Leu Glu Trp Ile
        35                  40                  45

Gly Gln Ile Trp Pro Gly Asp Gly Asp Thr Asn Tyr Asn Gly Lys Phe
    50                  55                  60

Lys Gly Lys Ala Thr Leu Thr Ala Asp Glu Ser Ser Ser Thr Ala Tyr
65                  70                  75                  80

Met Gln Leu Ser Ser Leu Ala Ser Glu Asp Ser Ala Val Tyr Phe Cys
                85                  90                  95

Ala Arg Arg Glu Thr Thr Thr Val Gly Arg Tyr Tyr Tyr Ala Met Asp
            100                 105                 110

Tyr Trp Gly Gln Gly Thr Thr Val Thr Val Ser Ser Gly Gly Gly Gly
        115                 120                 125

Ser Gly Gly Gly Gly Ser Gly Gly Gly Gly Ser Asp Ile Lys Leu Gln
    130                 135                 140

Gln Ser Gly Ala Glu Leu Ala Arg Pro Gly Ala Ser Val Lys Met Ser
145                 150                 155                 160

Cys Lys Thr Ser Gly Tyr Thr Phe Thr Arg Tyr Thr Met His Trp Val
                165                 170                 175

Lys Gln Arg Pro Gly Gln Gly Leu Glu Trp Ile Gly Tyr Ile Asn Pro
            180                 185                 190

Ser Arg Gly Tyr Thr Asn Tyr Asn Gln Lys Phe Lys Asp Lys Ala Thr
        195                 200                 205

Leu Thr Thr Asp Lys Ser Ser Ser Thr Ala Tyr Met Gln Leu Ser Ser
    210                 215                 220

Leu Thr Ser Glu Asp Ser Ala Val Tyr Tyr Cys Ala Arg Tyr Tyr Asp
225                 230                 235                 240

Asp His Tyr Cys Leu Asp Tyr Trp Gly Gln Gly Thr Thr Leu Thr Val
                245                 250                 255

Ser Ser Val Glu
            260

<210>  5
<211>  678
<212>  PRT
<213>  Artificial Sequence

<220>
<223>  recombinant antibody

<400>  5

Gln Ile Val Leu Thr Gln Ser Pro Ala Ile Met Ser Ala Ser Pro Gly
1               5                   10                  15

Glu Lys Val Thr Met Thr Cys Ser Ala Ser Ser Ser Val Ser Tyr Met
            20                  25                  30

Asn Trp Tyr Gln Gln Lys Ser Gly Thr Ser Pro Lys Arg Trp Ile Tyr
        35                  40                  45

Asp Thr Ser Lys Leu Ala Ser Gly Val Pro Ala His Phe Arg Gly Ser
    50                  55                  60

Gly Ser Gly Thr Ser Tyr Ser Leu Thr Ile Ser Gly Met Glu Ala Glu
65                  70                  75                  80

Asp Ala Ala Thr Tyr Tyr Cys Gln Gln Trp Ser Ser Asn Pro Phe Thr
                85                  90                  95

Phe Gly Ser Gly Thr Lys Leu Glu Ile Asn Arg Ala Asp Thr Ala Pro
            100                 105                 110

Thr Val Ser Ile Phe Pro Pro Ser Ser Glu Gln Leu Thr Ser Gly Gly
        115                 120                 125

Ala Ser Val Val Cys Phe Leu Asn Asn Phe Tyr Pro Lys Asp Ile Asn
    130                 135                 140

Val Lys Trp Lys Ile Asp Gly Ser Glu Arg Gln Asn Gly Val Leu Asn
145                 150                 155                 160

Ser Trp Thr Asp Gln Asp Ser Lys Asp Ser Thr Tyr Ser Met Ser Ser
                165                 170                 175

Thr Leu Thr Leu Thr Lys Asp Glu Tyr Glu Arg His Asn Ser Tyr Thr
            180                 185                 190

Cys Glu Ala Thr His Lys Thr Ser Thr Ser Pro Ile Val Lys Ser Phe
        195                 200                 205

Asn Arg Asn Glu Cys Gly Gly Gly Gly Ser Gly Gly Gly Gly Ser Gly
    210                 215                 220

Gly Gly Gly Ser Gln Val Gln Leu Gln Gln Ser Gly Ala Glu Leu Ala
225                 230                 235                 240

Arg Pro Gly Ala Ser Val Lys Met Ser Cys Lys Ala Ser Gly Tyr Thr
                245                 250                 255

Phe Thr Arg Tyr Thr Met His Trp Val Lys Gln Arg Pro Gly Gln Gly
            260                 265                 270

Leu Glu Trp Ile Gly Tyr Ile Asn Pro Ser Arg Gly Tyr Thr Asn Tyr
        275                 280                 285

Asn Gln Lys Phe Lys Asp Lys Ala Thr Leu Thr Thr Asp Lys Ser Ser
    290                 295                 300

Ser Thr Ala Tyr Met Gln Leu Ser Ser Leu Thr Ser Glu Asp Ser Ala
305                 310                 315                 320

Val Tyr Tyr Cys Ala Arg Tyr Tyr Asp Asp His Tyr Cys Leu Asp Tyr
                325                 330                 335

Trp Gly Gln Gly Thr Thr Leu Thr Val Ser Ser Ala Lys Thr Thr Ala
            340                 345                 350

Pro Ser Val Tyr Pro Leu Ala Pro Val Cys Gly Gly Thr Thr Gly Ser
        355                 360                 365

Ser Val Thr Leu Gly Cys Leu Val Lys Gly Tyr Phe Pro Glu Pro Val
    370                 375                 380

Thr Leu Thr Trp Asn Ser Gly Ser Leu Ser Ser Gly Val His Thr Phe
385                 390                 395                 400

Pro Ala Val Leu Gln Ser Asp Leu Tyr Thr Leu Ser Ser Ser Val Thr
                405                 410                 415

Val Thr Ser Ser Thr Trp Pro Ser Gln Ser Ile Thr Cys Asn Val Ala
            420                 425                 430

His Pro Ala Ser Ser Thr Lys Val Asp Lys Lys Ile Glu Pro Arg Pro
        435                 440                 445

Lys Ser Cys Asp Lys Thr His Thr Cys Pro Pro Cys Pro Ala Pro Glu
    450                 455                 460

Leu Leu Gly Gly Pro Ser Val Phe Leu Phe Pro Pro Lys Pro Lys Asp
465                 470                 475                 480

Thr Leu Met Ile Ser Arg Thr Pro Glu Val Thr Cys Val Val Val Asp
                485                 490                 495

Val Ser His Glu Asp Pro Glu Val Lys Phe Asn Trp Tyr Val Asp Gly
            500                 505                 510

Val Glu Val His Asn Ala Lys Thr Lys Pro Arg Glu Glu Gln Tyr Asn
        515                 520                 525

Ser Thr Tyr Arg Val Val Ser Val Leu Thr Val Leu His Gln Asp Trp
    530                 535                 540

Leu Asn Gly Lys Glu Tyr Lys Cys Lys Val Ser Asn Lys Ala Leu Pro
545                 550                 555                 560

Ala Pro Ile Glu Lys Thr Ile Ser Lys Ala Lys Gly Gln Pro Arg Glu
                565                 570                 575

Pro Gln Val Tyr Thr Leu Pro Pro Ser Arg Asp Glu Leu Thr Lys Asn
            580                 585                 590

Gln Val Ser Leu Thr Cys Leu Val Lys Gly Phe Tyr Pro Ser Asp Ile
        595                 600                 605

Ala Val Glu Trp Glu Ser Asn Gly Gln Pro Glu Asn Asn Tyr Lys Thr

610                 615                 620

Thr Pro Pro Val Leu Asp Ser Asp Gly Ser Phe Phe Leu Tyr Ser Lys
625                 630                 635                 640

Leu Thr Val Asp Lys Ser Arg Trp Gln Gln Gly Asn Val Phe Ser Cys
                645                 650                 655

Ser Val Met His Glu Ala Leu His Asn His Tyr Thr Gln Lys Ser Leu
            660                 665                 670

Ser Leu Ser Pro Gly Lys
        675

<210>  6
<211>  382
<212>  PRT
<213>  Artificial Sequence

<220>
<223>  fusion protein

<400>  6

Lys Gly Ile Phe Val Ile Gln Ser Glu Ser Leu Lys Lys Cys Ile Gln
1               5                   10                  15

Ala Gly Lys Ser Val Leu Thr Leu Glu Asn Cys Lys Gln Ala Asn Lys
            20                  25                  30

His Met Leu Trp Lys Trp Val Ser Asn His Gly Leu Phe Asn Ile Gly
        35                  40                  45

Gly Ser Gly Cys Leu Gly Leu Asn Phe Ser Ala Pro Glu Gln Pro Leu
    50                  55                  60

Ser Leu Tyr Glu Cys Asp Ser Thr Leu Val Ser Leu Arg Trp Arg Cys
65                  70                  75                  80

Asn Arg Lys Met Ile Thr Gly Pro Leu Gln Tyr Ser Val Gln Val Ala
                85                  90                  95

His Asp Asn Thr Val Val Ala Ser Arg Lys Tyr Ile His Lys Trp Ile
            100                 105                 110

Ser Tyr Gly Ser Gly Gly Gly Asp Ile Cys Glu Tyr Gly Gly Gly Gly
        115                 120                 125

Ser Gly Gly Gly Gly Ser Gly Gly Gly Gly Ser Asp Ile Lys Leu Gln
    130                 135                 140

Gln Ser Gly Ala Glu Leu Ala Arg Pro Gly Ala Ser Val Lys Met Ser
145                 150                 155                 160

Cys Lys Thr Ser Gly Tyr Thr Phe Thr Arg Tyr Thr Met His Trp Val
                165                 170                 175

Lys Gln Arg Pro Gly Gln Gly Leu Glu Trp Ile Gly Tyr Ile Asn Pro
            180                 185                 190

Ser Arg Gly Tyr Thr Asn Tyr Asn Gln Lys Phe Lys Asp Lys Ala Thr
        195                 200                 205

Leu Thr Thr Asp Lys Ser Ser Ser Thr Ala Tyr Met Gln Leu Ser Ser
    210                 215                 220

Leu Thr Ser Glu Asp Ser Ala Val Tyr Tyr Cys Ala Arg Tyr Tyr Asp
225                 230                 235                 240

Asp His Tyr Cys Leu Asp Tyr Trp Gly Gln Gly Thr Thr Leu Thr Val
                245                 250                 255

Ser Ser Val Glu Gly Gly Ser Gly Gly Ser Gly Gly Ser Gly Gly Ser
            260                 265                 270

Gly Gly Val Asp Asp Ile Gln Leu Thr Gln Ser Pro Ala Ile Met Ser
        275                 280                 285

Ala Ser Pro Gly Glu Lys Val Thr Met Thr Cys Arg Ala Ser Ser Ser
    290                 295                 300

Val Ser Tyr Met Asn Trp Tyr Gln Gln Lys Ser Gly Thr Ser Pro Lys
305                 310                 315                 320

Arg Trp Ile Tyr Asp Thr Ser Lys Val Ala Ser Gly Val Pro Tyr Arg
                325                 330                 335

Phe Ser Gly Ser Gly Ser Gly Thr Ser Tyr Ser Leu Thr Ile Ser Ser
            340                 345                 350

Met Glu Ala Glu Asp Ala Ala Thr Tyr Tyr Cys Gln Gln Trp Ser Ser
        355                 360                 365

Asn Pro Leu Thr Phe Gly Ala Gly Thr Lys Leu Glu Leu Lys
    370                 375                 380

<210>  7

<211>   1152
<212>   DNA
<213>   Artificial Sequence

<220>
<223>   fusion protein encoding sequence

<400>   7
aagggcatct tcgtgatcca gagcgaaagc ctgaaaaagt gcatccaggc cggaaaaagc        60

gtgctgacac tggagaactg caagcaggcc aacaagcaca tgctgtggaa atgggtgagc       120

aatcacggcc tgttcaacat cggcggcagc ggctgcctgg gcctgaactt ctccgccccc       180

gaacagcccc tgagcctgta cgagtgcgac agcaccctgg tgagcctgag atggagatgc       240

aacagaaaga tgatcacagg ccctctgcag tactccgtgc aggtggccca cgacaatacc       300

gtggtggcca gcagaaaata catccacaag tggatctctt acggcagcgg cggaggcgac       360

atctgcgagt acggcggagg cggcagcgga ggaggaggaa gtggaggcgg aggcagcgac       420

atcaagctgc agcagagcgg cgccgagctg gccagacccg gcgccagcgt gaagatgagc       480

tgcaagacca gcggctacac cttcaccaga tacaccatgc actgggtgaa gcagagaccc       540

ggccagggcc tggagtggat cggctacatc aaccccagca gaggctacac caactacaac       600

cagaagttca aggacaaggc caccctgacc accgacaaga gcagcagcac cgcctacatg       660

cagctgagca gcctgaccag cgaggacagc gccgtgtact actgcgccag atactacgac       720

gaccactact gcctggacta ctggggccag ggcaccaccc tgaccgtgag cagcgtggag       780

ggcggcagcg gcggcagcgg cggcagcggc ggcagcggcg gcgtggacga catccagctg       840

acacagagtc ccgctatcat gtctgccagc ccaggcgaaa aagtgactat gacctgcaga       900

gctagcagca gcgtgagcta catgaactgg taccagcaga agagcggcac cagccccaaa       960

agatggatct atgacacctc caaggtggcc tctggggtgc cttacagatt ctccggctcc      1020

ggcagcggca cctcctacag cctgactatc agcagcatgg aggccgagga cgccgccaca      1080

tactactgcc agcagtggag cagcaaccct ctgactttcg gcgccggcac caagctggaa      1140

ctgaagcacc at                                                          1152

<210>   8
<211>   318
<212>   PRT
<213>   Homo sapiens

<400>   8

Lys Gly Ile Phe Val Ile Gln Ser Glu Ser Leu Lys Lys Cys Ile Gln
1               5                   10                  15

Ala Gly Lys Ser Val Leu Thr Leu Glu Asn Cys Lys Gln Ala Asn Lys
            20                  25                  30

His Met Leu Trp Lys Trp Val Ser Asn His Gly Leu Phe Asn Ile Gly
        35                  40                  45

Gly Ser Gly Cys Leu Gly Leu Asn Phe Ser Ala Pro Glu Gln Pro Leu
    50                  55                  60

Ser Leu Tyr Glu Cys Asp Ser Thr Leu Val Ser Leu Arg Trp Arg Cys
65                  70                  75                  80

Asn Arg Lys Met Ile Thr Gly Pro Leu Gln Tyr Ser Val Gln Val Ala
                85                  90                  95

His Asp Asn Thr Val Val Ala Ser Arg Lys Tyr Ile His Lys Trp Ile
            100                 105                 110

Ser Tyr Gly Ser Gly Gly Gly Asp Ile Cys Glu Tyr Leu His Lys Asp
        115                 120                 125

Leu His Thr Ile Lys Gly Asn Thr His Gly Met Pro Cys Met Phe Pro
    130                 135                 140

Phe Gln Tyr Asn His Gln Trp His His Glu Cys Thr Arg Glu Gly Arg
145                 150                 155                 160

Glu Asp Asp Leu Leu Trp Cys Ala Thr Thr Ser Arg Tyr Glu Arg Asp
                165                 170                 175

Lys Trp Gly Phe Cys Pro Asp Pro Thr Ser Ala Glu Val Gly Cys Asp
            180                 185                 190

Thr Ile Trp Glu Lys Asp Leu Asn Ser His Ile Cys Tyr Gln Phe Asn
        195                 200                 205

Leu Leu Ser Ser Leu Ser Trp Ser Glu Ala His Ser Ser Cys Gln Met
    210                 215                 220

Gln Gly Gly Thr Leu Leu Ser Ile Thr Asp Glu Thr Glu Glu Asn Phe
225                 230                 235                 240

Ile Arg Glu His Met Ser Ser Lys Thr Val Glu Val Trp Met Gly Leu
                245                 250                 255

Asn Gln Leu Asp Glu His Ala Gly Trp Gln Trp Ser Asp Gly Thr Pro
            260                 265                 270

Leu Asn Tyr Leu Asn Trp Ser Pro Glu Val Asn Phe Glu Pro Phe Val
        275                 280                 285

Glu Asp His Cys Gly Thr Phe Ser Ser Phe Met Pro Ser Ala Trp Arg
    290                 295                 300

Ser Arg Asp Cys Glu Ser Thr Leu Pro Tyr Ile Cys Lys Lys
305                 310                 315

<210>  9
<211>  5
<212>  PRT
<213>  Artificial Sequence

<220>
<223>  peptide linker

<400>  9

Gly Gly Gly Gly Ser
1               5

<210>  10
<211>  10
<212>  PRT
<213>  Artificial Sequence

<220>
<223>  peptide linker

<400>  10

Gly Gly Gly Gly Ser Gly Gly Gly Gly Ser
1               5                   10

<210>  11
<211>  15
<212>  PRT
<213>  Artificial Sequence

<220>
<223>  peptide linker

<400>  11

Gly Gly Gly Gly Ser Gly Gly Gly Gly Ser Gly Gly Gly Gly Ser
1               5                   10                  15

<210>  12
<211>  20
<212>  PRT
<213>  Artificial Sequence

<220>
<223>  peptide linker

<400>  12

Gly Gly Gly Gly Ser Gly Gly Gly Gly Ser Gly Gly Gly Gly Ser Gly

1               5                   10                  15

Gly Gly Gly Ser
            20

<210>  13
<211>  6
<212>  PRT
<213>  Artificial Sequence

<220>
<223>  peptide linker

<400>  13

Gly Gly Gly Gly Gly Gly
1               5

<210>  14
<211>  7
<212>  PRT
<213>  Artificial Sequence

<220>
<223>  peptide linker

<400>  14

Gly Gly Gly Gly Gly Gly Gly
1               5

<210>  15
<211>  8
<212>  PRT
<213>  Artificial Sequence

<220>
<223>  peptide linker

<400>  15

Gly Gly Gly Gly Gly Gly Gly Gly
1               5